# EUROPÄISCHE PATENTSCHRIFT

(11) **EP 0 861 896 B1**
(45) Veröffentlichungstag und Bekanntmachung des Hinweises auf die Patenterteilung: **18.03.2009**
(21) Anmeldenummer: 98101338.6
(22) Anmeldetag: 27.01.1998
(51) Int. Cl.: C12N 15/54, C12N 9/12, C07K 16/40, C12Q 1/68, C12Q 1/48, G01N 33/573, A61K 31/70, A61K 38/45

(54) **Zellvolumenregulierte humane Kinase h-sgk**
Cell volume regulated human kinase h-sgk
Kinase humaine regulé par le volume de cellule (h-sgk)

(30) Priorität: 28.02.1997 DE 19708173
(43) Veröffentlichungstag der Anmeldung: 02.09.1998
(73) Patentinhaber: Lang, Florian, 72076 Tübingen (DE)
(72) Erfinder: Lang, Florian, Prof. Dr., 72076 Tübingen (DE); Waldegger, Siegfried, Dr., 72071 Tübingen (DE)
(74) Vertreter: Isenbruck, Günter

(56) Entgegenhaltungen:
- EP-A- 0 887 081
- WO-A-98/11234
- EMBL Database, Heidelberg, FRG Accession number EMHUM2:Y10032 6. Februar 1997 WALDEGGER, S.: "H. sapiens mRNA for putative serine/threonine protein kinase" XP002112098
- WEBSTER, M.K. ET AL.: "Characterization of sgk, a Novel Member of the Serine/Threonine Protein Kinase Gene Family Which Is Transcripionally Induced by Glucocorticoids and Serum" MOLECULAR AND CELL BIOLOGY, Bd. 13, Nr. 4, April 1993 (1993-04), Seiten 2031-2040, XP002054150
- WALDEGGER, S. ET AL.: "Cloning and characterization of a putative human serine/threonine protein kinase transcriptionally modified during anisotonic and isotonic alterations of cell volume" PROCEEDINGS OF THE NATIONAL ACADEMY OF SCIENCES OF USA, Bd. 94, Nr. 9, 29. April 1997 (1997-04-29), Seiten 4440-4445, XP002079929

## Beschreibung

Die vorliegende Erfindung betrifft die Klonierung und Charakterisierung einer humanen Serin/Threonin-Kinase (h-sgk: serum and glucocorticoid dependent kinase). Weiterhin betrifft die Erfindung Reagenzien für die Diagnostik von Zuständen, die mit einer Änderung des Zellvolumens und/oder des "macromolecular crowdings" im Körper einhergehen, wie beispielsweise Hypernatriämie, Hyponatriämie, Diabetes mellitus, Niereninsuffizienz, Hyperkatabolismus, hepatische Encephalopathie, Entzündungen und mikrobielle oder virale Infektionen. Die vorliegende Erfindung betrifft außerdem Arzneimittel, enthaltend die h-sgk, Nukleinsäuren, welche für die h-sgk kodieren oder Rezeptoren, insbesondere Antikörper, die spezifisch an die h-sgk binden.

Selbst bei konstanter extrazellulärer Osmolarität ist die Konstanz des Zellvolumens ständig durch Transport über Zellmembranen und zellulären Metabolismus, d.h. Bildung und Abbau osmotisch aktiver Substanzen gefährdet.

Zellschwellung und -schrumpfung stören das intrazelluläre Milieu durch Verdünnung bzw. Konzentrierung von zellulären Makromolekülen, die zu massiver Beeinträchtigung der zellulären Funktionen führen . Daher haben Zellen eine Vielzahl von Zellvolumen-regulierenden Mechanismen entwickelt. Zellschwellung führt in den meisten Geweben zu einer zellulären Abgabe von Ionen durch Aktivierung von Ionenkanälen und KCI-Cotransport. Zellschrumpfung führt umgekehrt zu zellulärer Aufnahme von Ionen durch Aktivierung des NaCl/KCl-Cotransporters und Na+/H+-Austauschers.

Darüberhinaus stimuliert Zellschrumpfung die zelluläre Akkumulierung und Zellschwellung die zelluläre Abgabe von Osmolyten, Molekülen, die speziell zur Erzeugung intrazellulärer Osmolarität eingesetzt werden [Burg, M. B. Am. J. Physiol. 268:F983-F996, 1995].

Schließlich beeinflussen Änderungen des Leberzellvolumens hepatozellulären Stoffwechsel und Genexpression [Häussinger et al. (1994) Am. J. Physiol. 267, E343-E355]. Zellschwellung wirkt wie ein anaboles Signal, das Protein- und Glykogensynthese stimuliert sowie Protein- und Glykogenabbau hemmt. Umgekehrt wirkt Zellschrumpfung als kataboles Signal, indem es den Abbau von Glykogen und Proteinen fördert und den Aufbau von Proteinen und Glykogen hemmt [Häussinger et al. (1994) Am. J. Physiol. 267, E343-E355].

Das Zellvolumen wurde als entscheidendes Element in der Regulation des Leberzellstoffwechsels durch Hormone, zelluläre Aminosäure-Aufnahme und oxidativen Stress erkannt.

Die Signalmechanismen, die die Zellfunktion an die Änderungen des zellulären Hydratationszustandes koppeln, sind weitgehend unbekannt. Änderungen des Zellvolumens erzielen ihre vielfältigen Wirkungen auf die Zellfunktion z.T. durch Stimulation oder Unterdrückung der Expression bestimmter Gene, deren Produkte dann die Expression oder Aktivität einer Vielzahl von Zellkomponenten beeinflussen. Um Gene aufzudecken, die bei Zellschwellung vermehrt exprimiert werden, führten wir einen differentiellen mRNA fingerprinting Assay an cDNAs aus Hepatocyten durch, die entweder isotoner oder anisotoner extrazellulärer Flüssigkeit ausgesetzt worden waren. Dabei wurden mehrere Banden erhalten, die bei Verwendung unterschiedlicher Primer differentielle Expressionsraten aufwiesen.

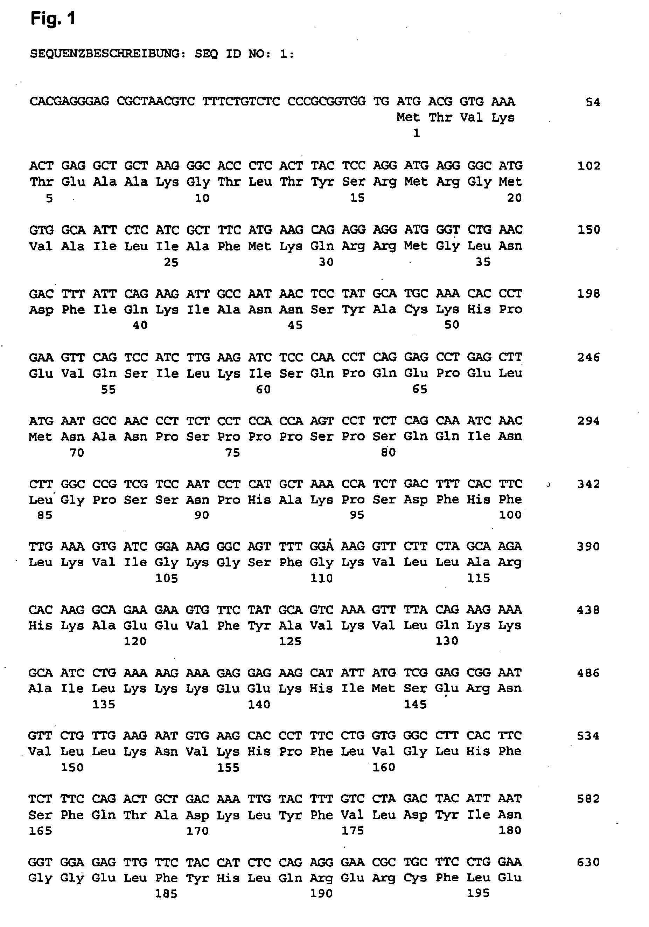

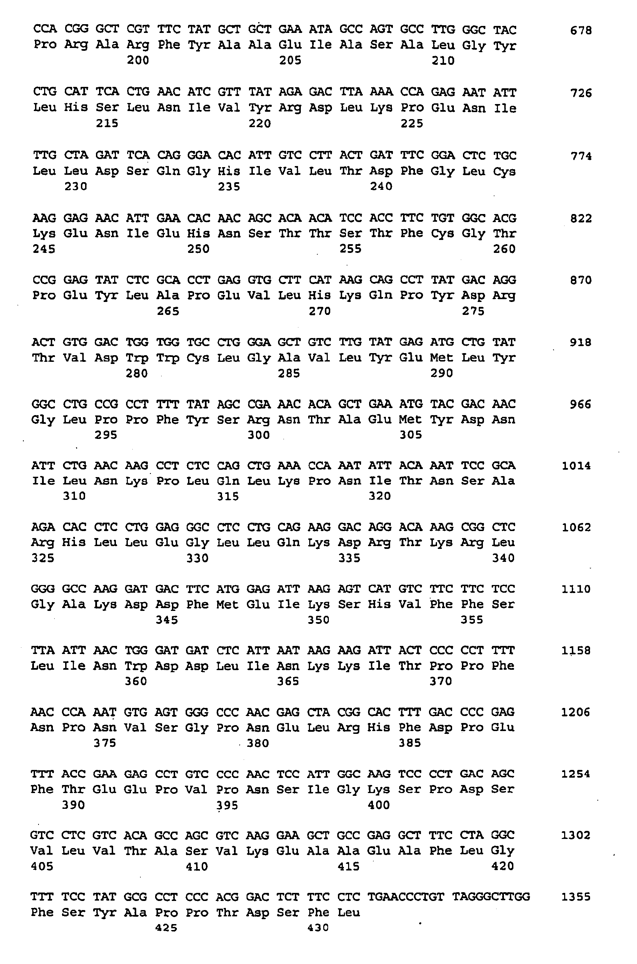

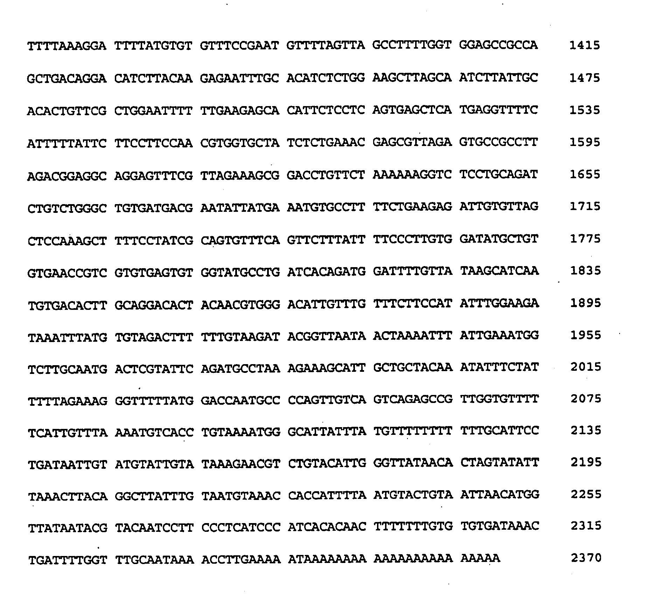

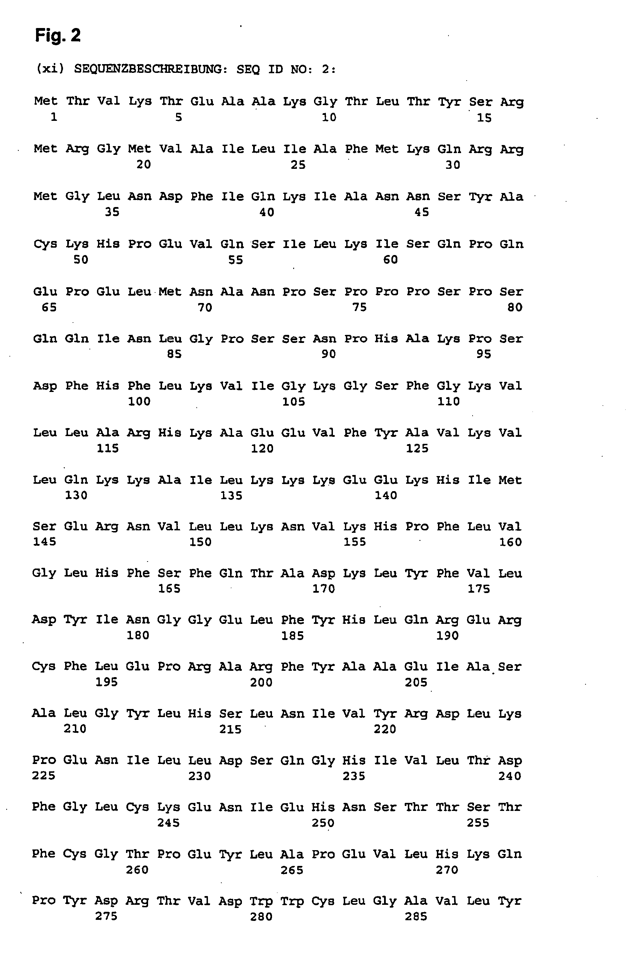

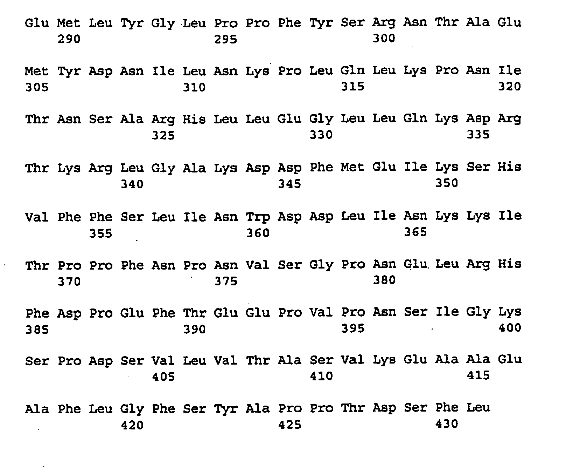

Es wurde überraschenderweise gefunden, daß die Expression einer dieser Banden unter hypertonen Bedingungen stimuliert und unter hypotonen Bedingungen gehemmt wurde. Die cDNA-Sequenz dieser in besonderer Weise durch Zellvolumenänderungen in ihrer Expression beeinflußten Bande, wurde genauer analysiert. Durch Sequenzvergleich wurde gefunden, daß keine Ähnlichkeit zu irgendeinem bereits bekannten menschlichen Gen gegeben ist. Das gefundene Gen, dessen Nukleotidsequenz in Fig. 1 dargestellt wird, kodiert überraschenderweise für eine Kinase, eine putative Serin-Threoninkinase. Ihre Sequenz ist in Fig. 2 sowie in Fig. 1 dargestellt. Sie ist hoch-homolog zur bereits bekannten Ratten-sgk (serum and glucocorticoid dependent kinase), einer Kinase, deren Expression durch Serum und Glucocorticoide gesteigert wird. Eine Zellvolumenabhängigkeit der Ratten-sgk ist bisher jedoch nicht beschrieben.

Datenbankeintrag Y10032 beschreibt die Sequenz der humanen zellvolumenregulierten Kinase h-sagk. Ferner wird erwähnt, dass die h-sgk durch Zellvolumenänderungen transkiptionell reguliert wird. Ein Zusammenhang mit Erkrankungen wird nicht dargestellt.

Die vorliegende Erfindung betrifft folglich ein Verfahren zum diagnostischen Nachweis mindestens einer der Erkrankungen aus der Gruppe Niereninsuffizienz, Entzündungen, insbesondere Hepatitis, Pankreatitis, Morbus Crohn oder Glomerulonephritis, Diabetes mellitus, mikrobielle oder virale Infektion, Morbus Alzheimer, Hyperkatabolismus, Lungenfibrose und Leberzirrhose, wobei das Verfahren das Bestimmen des Expressionsarades der humanen zellvolumenregulierten Kinase h-sgk oder des Expressionsgrades ihres Transkriptes umfasst, und wobei Diagnostizierverfahren ausgenommen sind, die am tierischen oder menschlichen Körper vorgenommen werden.

Die Expression der h-sgk ist in hohem Maße vom Zellvolumen abhängig. Zellschwellung hemmt die Expression der h-sgk, während Zellschrumpfung die Expression der h-sgk stimuliert. Darüberhinaus wird die Expression der h-sgk durch Harnstoff gehemmt. Harnstoff beeinträchtigt wie Zellvolumenänderungen die Stabilität und damit die Funktion zellulärer Proteine, bzw. die Packungsdichte der zellulären Makromoleküle, das sog. macromolecular crowding [Minton, A.P.,Mol. Cell. Biochem. 55: 119-140, 1983]. Die h-sgk-Expression ist daher ein Maß für das zelluläre "macromolecular crowding". Die Transskription der h-sgk wird jedoch, im Gegensatz zur Ratten-sgk, weder durch Corticoide noch durch fötales Kälberserum (FCS) beeinflußt.

Die h-sgk wird in einer Vielzahl menschlicher Gewebe exprimiert, wie Leber, Herz, Pankreas, Muskel, Niere, Lunge, Plazenta, Lymphocyten und mehreren Strukturen des Gehirns (Hippokampus, nucleus caudatus, corpus callosum, substantia nigra, nucleus subthalamicus und thalamus).

Es hat sich gezeigt, daß die h-sgk bei vielen Krankheiten, bei denen Zellvolumenänderungen eine entscheidende pathophysiologische Rolle spielen, ein beträchtliches diagnostisches Potential besitzt. Die Expression der h-sgk kann durch Nachweis und/ oder Quantifizierung der mRNA mit Hilfe geeigneter Probes, beispielsweise im Northem blot oder mittels in-Situ-Hybridisierung, und die h-sgk selbst kann beispielsweise mit Hilfe geeigneter Antikörper im Western blot oder durch Immunhistochemie nachgewiesen werden. Geeignete Probes bzw. Antikörper wurden bereits mit Erfolg auf Tauglichkeit überprüft.

Die vorliegende Erfindung betrifft daher auch die diagnostische Verwendung der h-sgk, ihrer Fragmente oder der jeweiligen dafür kodierenden Nukleinsäuren. Die möglichen zum Einsatz kommenden diagnostischen Techniken sind dem Fachmann bekannt. Es können dies alle aus dem Stand der Technik bekannten Immunoassayformate sein, wie beispielsweise Western blot oder Enzyme Linked Immunosorbent Assay (ELISA), aber auch homogene nicht festphasengebundene Assayformate. Denkbar sind beispielsweise kompetitive Testvarianten, aber auch indirekte Teste oder Aufbauten nach dem Sandwich-Prinzip sind ohne weiteres möglich. Ebenso können die dem Fachmann bekannten Markierungstechniken eingesetzt werden. Es können auch alle Arten der Nukleinsäurenachweistechniken angewandt werden, wie beispielsweise Southem blot, Northem blot und alle Varianten der Hybridisierungstechniken einschließlich In-Situ-Hybridisierung.

Die h-sgk kann sowohl in Körperflüssigkeiten, beispielsweise Blut, Plasma oder Serum, als auch in festen Geweben, beispielsweise Biopsiematerial nachgewiesen werden. Der Nachweis der h-sgk ist überall dort angezeigt, wo Änderungen des Zellvolumens bzw. des "macromolecular crowdings" im Körper auftreten, wie bei Hypernatriämie, Hyponatriämie, Diabetes mellitus, Niereninsuffizienz, Hyperkatabolismus, hepatischer Encephalopathie, Entündungen und Infektionen.

Darüberhinaus könnte eine gestörte Funktion der h-sgk zu einer gestörten Regulation des hepatischen Stoffwechsels führen. Ein Nachweis der h-sgk würde daher einer diagnostischen Abklärung von Fruktoseintoleranz, hyper- und hypoglykämischen Zuständen dienen.

**Hypernatriämie:** Dies ist eine lebensbedrohliche Störung, die beispielsweise bei osmotischer Diurese und Wasserdiurese durch zentralen oder nephrogenen Diabetes insipidus auftritt. Ein zentraler Diabetes insipidus ist Folge eines genetischen Defektes, Schädel-Hirn-Trauma, Schädigung der hypothalamischen Neurone durch Entzündungen, Mangeldurchblutung, Tumore, Konsum von Alkohol, Opiaten und einigen Pharmaka. Ein nephrogener Diabetes insipidus ist Folge von genetischen Defekten, Hypokaliämie, Hypercalcämie, Proteinmangel, Pyelonephritis, und Behandlung mit verschiedenen Pharmaka, etc.. Wie in Experimenten an kultivierten Leber- und Nierenzellen gezeigt wird, führt eine Zunahme der extrazellulären Na⁺ Konzentration, die immer auch mit einer Zunahme der extrazellulären Osmolarität verbunden ist, zu einer gesteigerten Expression der h-sgk. Die Kinase kann damit als Indikator für das Ausmaß an Zellschrumpfung herangezogen und zur Überprüfung der Therapie eingesetzt werden. Eine solche Kontrolle ist insofern bedeutsam, als bei zu schneller Korrektur einer Hypernatriämie trotz extrazellulärer Hyperosmolarität mitunter eine letale Zellschwellung auftreten kann.

**Hyponatriämie:** Bei etwa 1 - 2 % aller hospitalisierten Patienten stellt man eine Hyponatriämie unter 130 mmol/l fest. Ursachen dieser lebensbedrohlichen Störung sind Diabetes mellitus, Ketonurie, Leberinsuffizienz, Diuretica, Opiate, verschiedene Pharmaka, osmotische Diurese, Bikarbonaturie, Nebenniereninsuffizienz, Salzverlust-Niere (-Nephritis), Nephrotisches Syndrom, gesteigerte Ausschüttung von ADH und Verluste von isotoner Flüssigkeit (z.B. Durchfälle) mit ausschließlichem Ersatz von Wasser. Ist die Hyponatriämie Folge einer Zunahme anderer Osmolyte im Blut, dann bleiben Zellvolumen und Expression der h-sgk normal. Reflektiert die Hyponatriämie jedoch eine Hypoosmolarität mit Zellschwellung, dann ist die Expression der h-sgk herabgesetzt. Die Messung der h-sgk erlaubt somit Rückschlüsse auf das Vorliegen einer Zellschwellung und ermöglicht eine rationale Entscheidung über das therapeutische Vorgehen. Während der Therapie kann die Kinase zur Verlaufskontrolle eingesetzt werden. Die zu schnelle Korrektur einer Hyponatriämie kann zu mitunter letaler Zellschrumpfung führen.

**Diabetes mellitus**: Bei Diabetes mellitus kommt es zur Hyperglykämie, die zum Anstieg der extrazellulären Osmolarität führt und damit eine Zellschrumpfung hervorruft. Die glomerulär filtrierte Glucose übersteigt die maximale renale Transportrate und erzwingt auf diese Weise eine osmotische Diurese, bei der Na⁺ und Wasser verloren gehen. Dadurch kann sich eine Hyponatriämie entwickeln. Die gesteigerte extrazelluläre Osmolarität und das Überangebot an Glucose fördern die zelluläre Bildung von Sorbitol, das bei Absinken der extrazellulären Osmolarität zur Zellschwellung führt. Der Zellschrumpfung und der Zellschwellung bei Diabetes mellitus werden entscheidende Bedeutung bei der Pathophysiologie zugemessen [McManus et al.,New England J. Med. 333: 1260-1266, Dermadash et al.,Kidney intern 50:2032:2040, 1996]. Die Messung der h-sgk bei einem Patienten mit Diabetes mellitus erlaubt die Abschätzung der Zellvolumenänderungen und schafft damit eine solide Grundlage für den Ausgleich von Elektrolytstörungen. Auch dabei kann eine Verlaufsbeobachtung überschiessende Korrekturen verhindern.

**Niereninsuffizienz**: Bei der Niereninsuffizienz kommt es zu einer massiven Zunahme der Harnstoffkonzentration auf Werte, welche eine destabilisierende Wirkung auf Proteine ausüben, Zellen schrumpfen lassen und eine Abnahme der h-sgk -Expression bewirken. Die destabilisierende Wirkung von Harnstoff wird durch die Bildung von Trimethylaminen abgeschwächt. Bei schnellen Änderungen der Harnstoffkonzentration hält die Akkumulation von Trimethylaminen nicht Schritt, und es sind durch die Zellvolumenänderungen Störungen des Zellstoffwechsels zu erwarten. Die Bestimmung der h-sgk kann eine Imbalance zwischen destabilisierendem Harnstoff und stabilisierenden Trimethylaminen aufdecken. Gegebenenfalls wäre die therapeutische Applikation von Trimethylaminen bei stark unterdrückter h-sgk angezeigt.

**Hyperkatabolismus**: In einer Reihe kataboler Zustände, wie Sepsis, Verbrennungen, akuter Pankreatitis, schweren Operationen konnten Änderungen des Muskelzellvolumens nachgewiesen werden, die mit dem Ausmaß an Hyperkatabolismus korrelierten. Zellschrumpfung führt in der Tat zur Steigerung und Zellschwellung zur Hemmung der Proteolyse. Die Bestimmung der h-sgk könnte im Einzelfall den Einsatz von therapeutischen Maßnahmen rechtfertigen, die geeignet sind, der Zellschrumpfung entgegenzuwirken, wie die Applikation von Glutamin [Häussinger et al., Lancet, 341: 1330-1332, 1993] oder von Osmolyten [Burg, M.B.:J. Exp. Zool. 268(2):171-5, 1994].

**Hepatische Encephalopathie:** Es gibt überzeugende Beweise dafür, daß die hepatische Encephalopathie durch Schwellung von Gliazellen zustande kommt [Norenberg, M.D., Exp. Neurol. 53(3): 213-220, 1994]. Tatsächlich läßt sich bei Lebererkrankungen eine Abnahme des Osmolyten Inositol im Gehirn nachweisen [Kreis et al., NMR Biomed. 4: 109-116, 1991]. Das völlige Verschwinden koinzidiert mit dem Eintreten einer Encephalopathie. Durch Entwicklung und Einsatz geeigneter Substrate für die h-sgk könnte die h-sgk -Aktivität im Gehirn gemessen und bereits vor Auftreten der Encephalopathie gegengesteuert werden. Gegenenenfalls könnte auch die h-sgk -Expression in besser zugänglichen Geweben als Indikator für Volumenänderungen in Gliazellen herangezogen werden.

**Alzheimersche Krankheit**: Neue Hinweise deuten auf eine Zunahme des Volumens peripherer Zellen bei der Alzheimerschen Erkrankung hin. Darüberhinaus ist der Osmolyt Inositol bei Patienten mit Alzheimerscher Erkrankung erhöht, nicht jedoch bei einer Demenz anderer Genese. Die h-sgk-Expression kann somit zur Diagnose der Alzheimerschen Erkrankung beitragen.

**Infektionen/Entzündungen**: Bei einer Sepsis kommt es zu massiver Zellschrumpfung [Häussinger et al. , Lancet 1993, 341: 1330-1332] mit entsprechendem Auftreten von Hyperkatabolismus. Tatsächlich spielt beim Pathogen-Wirt-Verhältnis das Zellvolumen eine wichtige Rolle. Die Expression der h-sgk könnte ein wertvoller Parameter zur Abschätzung der Pathophysiologie von Infektionen sein. In-Situ-Hybridisierungsexperimente zeigen einen deutlichen Anstieg der Gewebekonzentration der h-sgk bei entzündlichen Erkrankungen, wie beispielsweise Hepatitis, Pankreatitis, Morbus Crohn oder Glomerulonephritis. Darüberhinaus wird die h-sgk-Expression durch TGFβ gesteigert, was mit einer progressiven Fibrose, wie beispielsweise Leberzirrhose, Lungenfibrose oder progressiver Niereninsuffizienz, in Zusammenhang gebracht wurde. Tatsächlich wurde gefunden, daß die h-sgk-Expression bei Patienten mit chronischer Niereninsuffizienz erhöht ist.

**Hyperglykämie-Hypoglykämie-Laktazidose**: Eine herabgesetzte oder gesteigerte Expression und/oder Funktion der h-sgk könnte zu Störungen des Kohlehydratstoffwechsels führen, wie sie bei Zellschrumpfung und Zellschwellung beobachtet wird [Lang et al., Pflügers Arch. 413: 209-216, 1989]. Eine herabgesetzte Funktion hätte zur Folge, daß eine Hypoglykämie droht. Eine gesteigerte Funktion könnte einerseits Hyperglykämie, andererseits Lactazidose nach sich ziehen. Bei der diagnostischen Abklärung von Hyperglykämie, Hypoglykämie und Lactacidosen unklarer Genese könnte daher immer auch die h-sgk Expression und Funktion untersucht werden.

Die vorliegende Erfindung wird außerdem durch die folgende detaillierte Beschreibung näher erläutert und darüberhinaus durch die Beispiele sowie die Patentansprüche beschrieben.

### Detaillierte Beschreibung der Erfindung

### Materialien und Methoden

**Materialien:** Fötales Kälberserum und DMEM (Dulbeccos modifiziertes Eagles Medium) wurden von GIBCO/BRL (Eggenstein, Deutschland) bezogen, Enzyme von STRATAGENE (Heidelberg, Deutschland) und BOEHRINGER MANNHEIM (Mannheim, Deutschland), a-[³⁵S]-dATP von ICN (Eschwege, Deutschland), SuperScript^{®} reverse Transcriptase von GIBCO/BRL. PCR^{®} (Polymerasekettenreaktionen) wurden in einem Crocodile^{®} II Thermocycler (APPLIGENE ONCOR, Heidelberg, Germany) mit Hilfe von Prime Zyme^{®} DNA polymerase und PCR Puffer von BIOMETRA (Göttingen, Deutschland) durchgeführt. RAP-PCR Primer wurden von STRATAGENE, Sequenzierprimer von MWG (Ebersberg, Deutschland) erworben. Manuelle Sequenzierung wurde auf einem S2-Sequenzierapparat von GIBCO/BRL mit Hilfe des Fidelity^{®} DNA Sequenzier-Systems (APPLIGENE ONCOR) durchgeführt.

**Zellkultur:** HepG2 humane Hepatomzellen wurden in Dulbeccos modifiziertem Eagles Medium (DMEM) mit 5 % CO₂, 5 mM Glucose bei 37°C, pH 7.4 kultiviert, das mit 10% (vol/vol) fötalen Kälberserum (FCS) angereichert war. Vor der RNA-Isolierung wurden die Zellen bis zu 90%iger Konfluenz kultiviert und für 12 Stunden in basalem Eagle Medium (BME, GIBCO/BRL) ohne fötalem Kälberserum gehalten. Die extrazelluläre Osmolarität wurde über Zugabe bzw. Wegnahme definierter Mengen von Kochsalz ohne Änderungen der anderen Komponenten des BME-Mediums variiert. In Experimenten, in denen die Wirkungen von Aminosäuren geprüft wurden, wurden die Zellen zwei Stunden vor Zugabe der Aminosäuren in einer aminosäurefreien extrazellulären Lösung gehalten.

**RAP-PCR:** RNA fingerprinting PCR (RAP-PCR) wurde wie früher beschrieben [McClelland et al. 1994, Nucleic Acids Res. 22, 4419-4431] durchgeführt. Nach Elektrophorese durch ein 4% Acrylamid / 7M Harnstoff Polyacrylamid-Gel, wurden die PCR-Produkte durch Silberfärbung sichtbar gemacht [Sanguinetti et al., Biotechniques 17, 914-921, 1994]. Alle Banden, die nur unter einer Bedingung (hyperton oder hypoton) sichtbar waren, wurden in der Folge durch reverse Transskription und PCR mit RNA aus neuen Kulturen bestätigt. Die RAP-PCR wurde mit vier verschiedenen Primerpaaren für die cDNA Synthese und PCR Amplifizierung durchgeführt. Zusätzlich wurden unterschiedliche Temperaturen zwischen 30°C und 40°C in der ersten Runde der Amplifizierung gewählt. Zusammen mit diesen Modifikationen wurden insgesamt 64 PCR-Durchgänge durchgeführt.

**Die Gewinnung der Banden**: Banden, die reproduzierbare Unterschiede zeigten, wurden unter sterilen Bedingungen ausgeschnitten. Das Amplicon wurde über Nacht bei 70 °C in 100 µl Puffer (50 mM KCl, 10mM TRIS-Cl pH 9.0, 0.1% Triton X 100) eluiert. Reamplifizierung durch PCR wurde mit 3.0 µl Eluat durchgeführt, mit geeigneten Primer (250 nM), 200 uM dNTP, 1x salzarmem Puffer (STRATAGENE) mit 1.5 mM MgCl₂ und 5 Einheiten Taq+^{®} DNA Polymerase (STRATAGENE) bei folgenden Temperatur-Zyklusprofilen: Ein Zyklus bei 95°C für 60 sec, 30 Zyklen bei 95 °C (15 sec), 55 °C (15 sec), 72 °C (60 sec) und zuletzt bei 72 °C für 5 Minuten. Nach Bestätigung durch PAGE, daß nur ein definiertes Amplicon mit der erwarteten Länge erzeugt worden war, wurde dieses Amplicon direct für die Bildung der Probe verwendet.

**Northern Analyse**: Digoxigenin (DIG)-gekoppelte Proben wurden durch direktes PCR-labeling der unterschiedlichen Amplicons erzeugt, mit Hilfe der geeigneten Primer und den Bedingungen, wie sie oben beschrieben wurden, außer, daß folgende dNTP Konzentrationen verwendet wurden: 200 µM dATP, 200 µM dCTP, 200 µM dGTP, 190 µM dTTP und 10 µM DIG-dUTP (BOEHRINGER). Northern blots wurden mit 20 µg totaler RNA oder mit 2 µg poly(A)-RNA hergestellt, die durch 1% Agarose Gele in Anwesenheit von 2.2M Formaldehyd elektrophoretisch getrennt worden waren. Äquivalentes Laden von Proben bei der Untersuchung von poly(A)-RNA wurde durch Färbung mit Ethidiumbromid der ribosomalen RNA Banden oder durch DIG-gelabelte Antisense-RNA probe gegen das humane heterogene nuclear ribonucleoprotein C1 als internem Standard überprüft. Die Größe der RNA wurde mit dem DIG-gelabelten Molekülgewicht-Marker l (BOEHRINGER) abgeschätzt. Vacuum blotting (APPLIGENE ONCOR Trans DNA Express Vacuum Blotter) wurde zum Transfer auf positiv geladene Nylonmembranen (BOEHRINGER) eigesetzt, die dann durch Ultraviolett-Licht vernetzt wurden (STRATAGENE UV Stratatinker^{®} 2400). Hybridisierung wurde über Nacht in DIG-Easy-Hyb^{®} (BOEHRINGER) bei einer Probenkonzentration von 25 ng/ml oder 100 ng/ml bei 50°C oder 65°C für DNA - Proben oder RNA - Proben durchgeführt. Proben, die unterschiedliche Expression zeigten, wurden mit dem pCR-Script SK(+) Cloning Kit (STRATAGENE) subkloniert und in Northem blots überprüft. Die im folgenden gezeigten Northem blots wurden von diesen Subclones abgeleitet.

**Weitere Methoden**: DNA Sequenzierung vom pCR clone wurde mit dem Fidelity^{®} DNA Sequencing System (APPLIGENE ONCOR) durchgeführt. Sequenzierungsprodukte wurden mit a-[³⁵S]-dATP markiert und auf einem 6% Polyacrylamide / 8M Harnstoff Sequenzierungsgel aufgetrennt. Die GenBank-Daten wurden nach homologen Sequenzen mit dem FASTA computer program [Pearson,W.R. & Lipman, D.J. (1988) Proc. Natl. Acad. Sci. USA 85, 2444-2448] abgesucht. Die Sequenz der vollständigen h-sgk cDNA wurde mit Hilfe des I.M.A.G.E. Consortium Clone ID 42669 von der TIGR/ATCC Special Collection of Human cDNA Clones gewonnen. Die Gendaten wurden mit Hilfe des European Molecular Biology Laboratory EMBL (Heidelberg), dem BLAST network service und - für die Protein alignments - dem BLITZ server an der neuesten Ausgabe der SwissProt protein database untersucht.

**Nucleotidsequenz-Zugangsnummer**: Die h-sgk cDNA-Sequenz wurde in die GenBank-Database unter der Zugangsnummer Y10032 eingegeben und bis zum 1. März 1997 gesperrt.

### Ergebnisse

**Differentielle Genexpression in HepG2-Zellen in hypotoner, isotoner und hypertoner extrazellulärer Flüssigkeit:** mRNA wurde von HepG2 Zellen isoliert, die für 1 oder 2 Stunden mit hypotoner (hypoton I: minus 100 mosmol/l durch Entfernung von 50 mM NaCl und hypoton II: minus 50 mosmol/l durch Entfernung von 25 mM NaCl im Vergleich mit isotonem Kontrollmedium), isotoner (mit gesamter Osmolarität von 290 mosmol/l und einer NaCl - Konzentration von 114 mM) oder hypertoner (plus 50 mosmol/l durch Zugabe von 50 mM Raffinose) Medium vorbehandelt wurden. Die mRNA wurde als Template für die RAP - PCR mit arbiträren Primern verwendet. Die Produkte der RAP - PCR wurden auf denaturierten Polyacrylamid-Gelen aufgetragen und zum Vergleich in parallelen Bahnen aufgetrennt. Mehrere Banden zeigten differentielle Expression bei Verwendung mehrerer Primer. Vier differentielle Banden der RAP-PCR Gele wurden weiter analysiert: Zwei erwiesen sich bei der folgenden Northem blot Analyse als falsch positiv, eine Bande wurde durch hypotone und hypertone Bedingungen gesteigert, aber ihre Sequenz zeigte keine Ähnlichkeit zu irgend einem bisher bekannten klonierten Gen. Eine Bande von etwa 500 Basenpaaren zeigte eine zunehmende Expression mit zunehmender extrazellulärer Osmolarität (hypoton I- hypoton II - isoton - hyperton). Diese Bande wurde aus dem Gel gereinigt und mit Hilfe von Primer RAP-A4 reamplifiziert. Nach Markierung mit Digoxigenin durch PCR, wurden Northern blots mit diesem Amplicon angefertigt, um die differentielle Expression in verschiedenen Zellpräparationen zu bestätigen, die für zwei Stunden mit hypotonem I, isotonem und hypertonem Medium vorbehandelt worden waren.

Ein einzelnes Transcript von etwa 2.6 Kilobasen war in hohem Maße durch die Änderungen der extrazellulären Osmolarität beeinflußt (Abb. 1). Die Transkriptmenge wurde bei Abnahme der Osmolarität gesenkt und bei Zunahme der Osmolarität gesteigert.

**Klonierung und Sequenzierung des differentiell regulierten h-sgk Gens.** Das 500 Basenpaare lange PCR-Produkt wurde in den PCR II Vektor subkloniert und eine neue Probe mit diesem Konstrukt erzeugt, um eine Identität zwischen dem ursprünglichen und dem subklonierten DNA-Fragment nachzuweisen. Rehybridisierung eines Northem blots mit dieser Probe führte zu identischen Ergebnissen wie die ursprüngliche Probe. Zusätzlich wurde eine Southern blot - Analyse mit dem neuen Konstrukt durchgeführt und mit dem ursprünglichen Konstrukt hybridisiert. Eine starke Hybridisierung nach zwei high stringency washes bestätigte die Identität der Sequenz.

Sequenzanalysen in beide Richtungen zeigten die Anwesenheit der verwendeten Primer an beiden Seiten des Amplicons. Eine Aminosäuresequenz, die von einem Leserahmen der Nucleotidsequenz translatiert wurde, zeigte eine 95 %ige Identität zur carboxyterminalen Aminosäuresequenz der Ratten-sgk (serum and glucocorticoid regulated protein kinase), einem neuen Mitglied der Serin/Threonin-Proteinkinase Proteinfamilie, das aus einer Ratten- Brustdrüsen-Tumorzellinie geklont wurde [Webster et al., Mol. Cell. Biol. 13, 2031-2040, 1993a]. Wegen der hohen Ähnlichkeit wurde die Bezeichnung h-sgk (human-) für das neue Protein gewählt.

Die Genbank-Database wurde auf ähnliche menschliche Sequenzen mit dem FASTA Computerprogramm durchsucht. Mehrere EST (Expressed Sequence Tags) DNA Sequenzen aus der TIGR/ATCC Special Collection of Human cDNA Clones zeigten 100 % Sequenzübereinstimmung mit Teilen der h-sgk cDNA Fragmente. Nach vielfachen alignments von 30 verschiedenen TIGR/ATCC humanen cDNA Klonen mit der Ratten-sgkcDNA-Sequenz (Genbank Zugangsnummer L01624) und mit dem h-sgk DNA Fragment, wurde angenommen, daß das I.M.A.G.E. Consortium Konstrukt mit dem Clone ID 42669 einer menschlichen Kinderhirn-Library die vollständige kodierende Sequenz der h-sgk aufweist. Sequenzanalyse dieses Konstruktes mit übereinstimmenden Sequenzen in Sense- and Antisense-Richtung ergab eine cDNA Sequenz von etwa 2.4 Kilobasen. Um die Beteiligung der vollständigen h-sgk nachzuweisen, wurde das 5' Ende des Klons (Nucleotide 1 - 285 der kodierenden Sequenz) in den pCR II vector subkloniert und damit eine neue Probe mit diesem Konstrukt erzeugt. Hybridisierung eines Northern blots mit dieser Probe resultierte in identischen Ergebnissen wie die ursprünglichen Proben (Abb. 1). Der längste Leserahmen des untersuchten Klons (1.3 kb) ergab ein 431-Aminosäure-Protein mit einer Identität von insgesamt 98 % zu dem Ratten-sgk Protein.

**Regulation der h-sgk Expression durch Änderungen der extrazellulären Osmolarität:** Um den Einfluß von Änderungen der extrazellulären Osmolarität auf die h-sgk-Transskriptmengen zu untersuchen, wurden HepG2 Zellen unterschiedlich lang in hypotonem (190 mol/l), isotonem (290 mosmol/l) und hypertonem (390 mosmol/l) BME Medium ohne FCS inkubiert. Die h-sgk mRNA Konzentrationen nahmen binnen 60 min in hypertoner Lösung stark zu. Der erste Anstieg war innerhalb von 30 Minuten erkennbar und erreichte ein Maximum innerhalb von zwei Stunden. Die Induktion der h-sgk folgt daher unmittelbar nach Änderung der Osmolarität. Die Transskriptkonzentrationen stiegen binnen 4 bis 8 Stunden in hypertonem extrazellulären BME-Medium weiter an, und fielen dann allmählich wieder innerhalb der folgenden 16 bis 24 Stunden auf die Ausgangskonzentrationen ab. Andererseits fielen die h-sgk-Transskriptkonzentrationen in hypotoner extrazellullärer Lösung schnell ab, wobei der Abfall bereits nach 30 Minuten erkennbar war und ein Maximum innerhalb von zwei Stunden erreicht wurde.

Unterschiedliche Osmolaritäten (140, 190, 240, 290, 340, 390 und 440 mosmol/l) zeigten binnen zwei Stunden unterschiedliche Expressionen der h-sgk. Über dem gesamten Bereich war eine steile Korrelation der h-sgk Expression mit der extrazellulären Osmolarität nachweisbar. Eine 30%ige Zunahme der Osmolarität ging in etwa mit einer Verdreifachung der Kinaseexpression einher. Eine Zunahme der Osmolarität von 290 auf 340 mosmol/l, und eine Abnahme der Konzentration von 290 auf 240 mosmol/l induzierten signifikante Änderungen der h-sgk Expression. Der transskriptionelle Kontrollmechanismus reagiert also offenbar sehr empfindlich auf Änderungen der Osmolarität. Die Induktion der h-sgk-RNA war unabhängig von einer de novo Proteinsynthese. Der Anstieg der Transskriptkonzentration in hypertonem BME Medium ist in Anwesenheit des Proteinsynthesehemmers Cycloheximid (10µg/ml ) größer als in Abwesenheit des Hemmstoffes.

Die schnelle Abnahme der h-sgk Transskriptkonzentrationen gleich nach Senkung der extrazellulären Osmolarität legt eine kurze Halbwertszeit der h-sgk-mRNA nahe. Um die Abnahmerate der h-sgk Transcriptkonzentrationen zu untersuchen, wurden HepG2 Zellen für zwei Stunden mit hypertonem Medium (390 mosmol/l) behandelt, um einen maximalen Anstieg der h-sgk Transskriptkonzentrationen zu erzielen. Dann wurde ein Teil der Zellen dem RNA-Polymerasehemmer Actinomycin D (5µg/ml) ausgesetzt, der andere Teil der Zellen hypotonem Medium (190 mosmol/l). Nach verschiedenen Zeitpunkten wurde RNA präpariert und die Transskriptkonzentrationen der beiden Zellgruppen verglichen. Actinomycin D Behandlung führte zu einem schnellen Abfall der h-sgk Transskriptkonzentrationen mit einer geschätzten Halbwertszeit von etwa 30 Minuten. Behandlung der Zellen mit hypotonem extrazellulären Medium führte zu einem gleichermaßen schnellen Abfall der Transskriptkonzentrationen.

**Regulation der h-sgk Transskriptkonzentrationen durch isotone Zellvolumen-Änderungen.** Um zwischen den Wirkungen von Änderungen des Zellvolumens, der Ionenstärke und der Osmolarität unterscheiden zu können, wurde das Zellvolumen durch zwei verschiedene Methoden bei gleichbleibender Ionenstärke und Osmolarität manipuliert. Binnen zwei Stunden isotoner Zellschrumpfung durch Hemmung des NaCl/KCl-Kotransporters und des Na+ /H+ -Austauschers mit Bumetanid und 3-Methylsulfonyl-4-(1-piperidino)-benzoylguanidin (EP-0 416 499) war die Expression der h-sgk gesteigert, eine Wirkung, die durch zusätzliche Zunahme der extrazellulären Osmolarität noch verstärkt wurde. Zellschwellung durch Angebot von verschiedenen Aminosäuren (Aminosäure-Mischung von 1x BME Aminosäuren, GIBCO/BRL) führte umgekehrt binnen zwei Stunden zu einem Abfall der h-sgk-Transskript-Konzentrationen. Es ist also das Zellvolumen, nicht die Osmolarität oder die Ionenstärke, welches die Expression der h-sgk reguliert.

Um zu prüfen, ob die Expression der h-sgk in HepG2 Zellen, ähnlich wie die der Ratten-sgk in Brustdrüsentumorzellen [Webster, M.K. (1993) Mol. Cell. Biol. 13, 2031-2040.] durch Glucocorticoide oder fötales Kälberserum (FCS) reguliert wird, wurden HepG2 Zellen mit Dexamethason (1µM) oder mit FCS (10 %) für zwei bis 12 Stunden inkubiert. In Northern blots konnte kein Einfluß von Glucocorticoiden oder FCS auf die Transskript-Konzentrationen von h-sgk in HepG2-Zellen gefunden.

**Regulation der sgk Transskriptkonzentrationen durch die extrazelluläre Osmolarität in Madin Darby Canine Kidney (MDCK) Zellen.** Um zu prüfen, ob die beobachtete Zellvolumen-Abhängigkeit der Expression von h-sgk eine Besonderheit der HepG2-Zellen ist, wurden die Hunde-Nierenepithelzellen MDCK für zwei Stunden hypotonem (190 mosmol/l) und hypertonem (390 mosmol/l) BME Medium ausgesetzt. h-sgk-Transskripte mit einer Länge von etwa 2.6 Kilobasen konnten auch nach mehreren High-Stringency-Waschschritten mit 0.5 x SSC (standard saline citrate) bei 65°C nachgewiesen werden, ein Hinweis auf hohe Sequenzhomologie der sgk-Gene zwischen verschiedenen Species. Änderungen der extrazellulären Osmolarität übten in MDCK-Zellen einen ähnlichen Einfluß auf die Transskriptkonzentrationen aus wie in HepG2-Zellen.

**Gewebsspezifische Expression der h-sgk.** Ein vorgefertigter Multiple Tissue Northern Blot (CLONTECH, Heidelberg, Germany) wurde mit der h-sgk DNA Probe untersucht. Die h-sgk Expression zeigt eine gewisse Gewebsspezifität, mit höchster Expression in Pancreas, Leber, und Herzmuskel. Eine etwas geringere Expression zeigt sich in Placenta, Lunge und Skelettmuskel. Niedrige aber erkennbare Expressionen finden sich in Gehirn und Niere. In menschlichem Gehirngewebe ist die Expression in der substantia nigra und dem corpus callosum am höchsten, in den corpora amygdala, Hippocampus, nucleus caudatus und nucleus subthalamicus durchschnittlich und im Thalamus am geringsten. Interessanterweise wurde ein zweites Transskript von 7 Kilobasen in fast allen Geweben gefunden, mit höchster Expression im Pancreas. Dieses Transskript ist möglicherweise eine weitere h-sgk-mRNA durch alternatives splicing oder ein zur h-sgk homologes Gen. Das 7 Kilobasen-Transcript war in HepG2 Northern blots nicht gefunden worden.

**Regulation der h-sgk Expression durch Harnstoff**: Die h-sgk-Expression ist durch die Anwesenheit von Harnstoff im Extrazellulärraum unterdrückt. Die Expression der h-sgk war bei 50mmol/l Harnstoff mässig, bei 100 mmol/l Harnstoff massiv eingeschränkt.

### Diskussion

Die h-sgk, ein humanes Gen, dessen Transskription durch Änderungen des Zellvolumens reguliert wird, kodiert für eine putative Serin/Threonin-Proteinkinase mit hoher Sequenzhomologie zu Ratten-sgk, die kürzlich als Serum- und Glucocorticoid-reguliertes Gen von Ratten-BrustdrüsenTumorzellen, als Läsions-induziertes Gen nach ZNS- Läsionen im Rattenhirn [Imaizumi et al., Mol. Brain Res. 26, 189-196, 1994] und als Testosteron- und Follikel-stimulierendes Hormon- induziertes Gen in Granulosa-Zellen des Ratten-Ovars [Richards et al., Recent Prog. Horm. Res. 50, 223-254, 1995] beschrieben wurde. Das 49 kD h-sgk Protein zeigt annähernd 98 % Homologie mit dem Ratten sgk Protein mit weitgehend konservativen Aminosäureaustauschen. Es weist etwa 50% Homologie in seiner katalytischen Domäne mit mehreren Kinasen der "second messenger" Familie auf, wie rac Protein-kinase, Protein kinase C, ribosomale protein S6 kinase, and cAMP-abhängige Protein kinase [Webster et al., (1993b) J. Biol. Chem. 268, 11482-11485,Webster et al., (1993a) Mol. Cell. Biol. 13, 2031-2040].

Der Expressionsgrad des 2.6 Kilobasen h-sgk Transkripts in HepG2 Zellen wird in hohem Maße durch Änderungen der extrazellulären Osmolarität beeinflußt. Gesteigerte Transkript-Konzentrationen konnten binnen 30 Minuten nach Zunahme der extrazellulären Osmolarität gefunden werden. Diese Induktion war unabhängig von einer de novo Protein-Synthese. Die Transkriptkonzentrationen sinken binnen 30 Minuten nach Senkung der extrazellulären Osmolarität. Der Abfall war gleichermassen schnell wie der Abfall nach Hemmung der Transkription durch Aktinomycin D.

Zellvolumenänderungen beeinflussen demnach die Transskriptionsrate von h-sgk. Die herabgesetzte h-sgk Transskriptionsrate nach osmotischer Zellschwellung und die kurze Halbwertszeit gewährleisten eine schnelle und effiziente Regulation der Transskript-Konzentrationen von h-sgk RNA in HepG2 Zellen.

Isosmotische Änderungen des Zellvolumens beeinflussen gleichermassen die h-sgk-Expression. Zellschrumpfung wurde durch Hemmung der wesentlichen Ionentransportmechanismen Na⁺ / H**⁺** -Austauscher und NaCl / KCl - Cotransporter durch deren spezifische Blocker 3-Methylsulfonyl-4-(1-piperidino)-benzoylguanidin (EP-0 416 499) und Bumetanid erreicht. Zellschwellung wurde durch die Zugabe von Aminosäuren und die folgende hepatozelluläre Akkumulierung der Aminosäuren über Na⁺ - abhängige Aminosäuretransporter wie etwa System A, N, und ASC bewirkt. Die Transskriptkonzentrationen korrelierten mit dem Zellvolumen, nicht mit der Osmolarität.

Nach langanhaltender osmotischer Zellschrumpfung nahmen die Transskriptkonzentrationen innerhalb der ersten halben Stunde steil zu und blieben dann für 8 Stunden erhöht, bevor sie wieder allmählich abnahmen. Diese langanhaltende Zunahme steht im scheinbaren Widerspruch zum schnellen Zeitgang der Zellvolumenregulation. Osmotisch geschrumpfte oder geschwollene Leberzellen regulieren ihr Zellvolumen jedoch nicht völlig, sondern bleiben nach der schnellen Phase der Volumenregulation noch mässig geschrumpft bzw. geschwollen [Häussinger et al.,. (1994) Am. J. Physiol. 267, E343-E355].

Die verbleibenden Zellvolumenänderungen könnten für die veränderte Expression der h-sgk verantwortlich sein.

Neben dem Zellvolumen selbst übt Harnstoff einen massiven Einfluß auf die Expression der h-sgk aus. Harnstoff übt eine destabilisierende Wirkung auf Proteine aus und imitiert auf diese Weise die Wirkung einer Zellschwellung. Die h-sgk wäre somit ein Sensor der Proteinstabilität bzw. der Packungsdichte der zellulären Makromoleküle. Die destabilisierende Wirkung von Harnstoff wird durch die Bildung von Trimethylaminen abgeschwächt, wodurch wahrscheinlich die destabilisierende Wirkung von Harnstoff bei Niereninsuffizienz abgeschwächt wird.

Die zellulären Wirkungen der h-sgk sind noch unsicher. Insbesondere läßt sich derzeit noch nicht mit Sicherheit feststellen, ob die Wirkungen der h-sgk eine Rolle für die Zellvolumenregulation spielen. Für einen Einsatz der h-sgk als Diagnostikum ist die Wirkung der h-sgk jedoch belanglos.

Trotz der auffälligen Sequenzhomologie zur Ratten-sgk konnten wir keine Parallelen zur Regulation der Ratten-sgk feststellen. Weder Serum (FCS) noch Glucocorticoide (Dexamethason), die beide in Ratten-BrustdrüsenTumorzellen einen starken Einfluß auf die sgk Transcription gezeigt hatten, beeinflußten die Expression von h-sgk in HepG2 Zellen. In den verschiedenen Zelltypen scheinen daher unterschiedliche h-sgk Promotersequenzen die Expression des Proteins zu regulieren. Es ist also denkbar, daß die Expression der h-sgk nicht ausschließlich durch das Zellvolumen bzw. die Packungsdichte reguliert wird. Eine Zellvolumen-Abhängigkeit der h-sgk Expression konnten wir auch in Nierenepithelzellen (MDCK) und in Makrophagen nachweisen. Die Zellvolumenabhängigkeit der h-sgk-Expression ist also keine Besonderheit der HepG2 Zellen. Die h-sgk 5'flankierenden Sequenzen in den verschiedenen Zellen könnten die regulierenden Elemente aufdecken, die für die unterschiedlioche Expression der sgk Transskripte verantwortlich sind. Wie die früher beschriebene Glucocorticoid- und Serum induzierte Expression von sgk in der Ratte benötigt die Zellvolumen induzierte Expression der h-sgk RNA nur 30 Minuten. Die Halbwertszeit der h-sgk Transskripte in HepG2 Zellen ist mit 30 Minuten ebenso kurz wie die sgk Halbwertszeit in Ratten-Brustdrüsentumorzellen, wie die Experimente mit dem RNA-Polymerasehemmer Actinomycin D zeigen.

Das h-sgk-Transskript wird in allen bisher untersuchten menschlichen Geweben exprimiert. Besonders hoch ist die Expression in Pancreas und Leber, möglicherweise aufgrund der spezialisierten Epithelfunktion dieser Gewebe.

Proteinphosphorylierung ist ein schneller und reversibler Mechanismus, um Signale aus dem Extrazellulärraum in die Änderungen von einer Vielzahl von Zellfunktionen umzusetzen. Die h-sgk-Proteinkinase könnte durch Phosphorylierung spezifischer Proteine einen Teil der zellvolumenregulatorischen Mechanismen auslösen und ein bisher unbekanntes Bindeglied zwischen zellulärer Hydratation und Zellfunktion darstellen.

### Beispiele:

### 1. Durchführung der Northern Hybridisierungen:

10-20 mg Gesamt-RNA bzw. 1-2 mg poly(A)-RNA wurden elektrophoretisch in einem 1%igen Agarosegel in Gegenwart von 2.2M Formaldehyd aufgetrennt. Der Transfer auf eine positiv geladene Nylonmembran erfolgte mit Hilfe eines Vacuum Blotters mit 10x SSC als Transferpuffer für einen Zeitraum von zwei Stunden. Anschließend wurde die RNA durch UV-Bestrahlung mit kontrollierter Leistung kovalent an die Membran quervernetzt. Die Hybridisierung der spezifischen Sonde (25ng/ml) wurde bei 50°C über Nacht durchgeführt in einem speziell für den Zweck der nichtradioaktiven Hybridisierung entwickelten Puffer (DIG Easy Hyb, BOEHRINGER). Die dabei verwendete Sonde wurde mittels der Polymerasen Ketten Reaktion aus dem 3*'*Ende der kodierenden Sequenz der betreffenden h-sgk (Nucleotid 980-1480) amplifiziert und gleichzeitig durch Einschluß von DIG-dUTP im Reaktionspuffer markiert. Nach zweimaligem Waschen der Blots in 2x SSC bei Raumtemperatur und in 0.5x SSC bei 65°C erfolgte die Detektion der markierten Sonde durch einen ELISA mit einem Anti-Digoxigenin Antikörper, dessen angekoppelte alkalische Phosphatase eine Chemolumineszenzreaktion in CDP-Star (BOEHRINGER) erzeugte, die autoradiographisch festgehalten wurde (durchschnittliche Expositionszeit ca. zwei Minuten).

### 2. Western Blot Analyse:

**Details zur Antikörperherstellung:** Zur Immunisierung der Kaninchen wurden zwei Peptide aus der h-sgk-Aminosäurensequenz verwendet: Pos.386-Pos.404 (DPEFTEEPVPNSIGKSPDS), Pos.416-Pos.431 (EAFLGFSYAPPTDSFL). Beide Peptide wurden mit KLH bzw. mit MAP als Carrier konjugiert und mit komplettem bzw. inkomplettem Freund*'*schen Adjuvans intracutan injiziert. Das Injektions- und Blutungsprotokoll folgte den Standardvorschriften. Die Immunseren wurden affinitätschromatographisch gereinigt, und die Antikörperfraktionen wurden bei einer Konzentration von ca. 1 mg/ml aufgefangen und verwendet.

**Immunoblot-Analyse:** Ca. 60mg zelluläres Gesamtprotein wurden elektrophoretisch durch ein SDS-7.5% Polyacrylamid Gel fraktioniert und auf eine Nitrozellulose-Membran transferiert. Die Membranen wurden über Nacht in 3% BSA - 5% Trockenmilch - 0.06% Tween 20 in PBS blockiert. Primärer (affinitätsgereinigter anti-h-sgk) und sekundärer (horseradish-Peroxidase konjugierter goat anti-rabbit IgG, Bio-Rad) wurden für je eine Stunde bei Raumtemperatur in 3% BSA-0.06% Tween in PBS inkubiert. Für die Immunodetektion wurde ein Enhanced Chemolumineszenz Kit (ECL, Amersham) verwendet.

### 3. In Situ-Hybridisierung:

15 mm Gefrierschnitte wurden für 20 Minuten in Formaldehyd 4% nachfixiert, gefolgt von zwei Waschschritten in 100mM Phosphatpuffer pH 7.2 von jeweils 5 Minuten. Nach einer Proteinase K Behandlung (1mg/100ml) folgte eine 10 minütige Inkubation in 0.1 M Triethanolamin / 0.225% Essigsäure. Erneut wurde mit 100 mM Phosphatpuffer gewaschen und die Schnitte in einer aufsteigenden Alkoholreihe entwässert. Die Prähybridisierung erfolgte bei 50 °C im Hybridisierungspuffer, die Hybridisierung wurde über Nacht durchgeführt. Die verwendete Sonde entspricht der Sonde, die bei den Northem Blots beschrieben wurde. Die Detektion erfogte durch enzymatische Spaltung einer X-Phosphatlösung, katalysiert durch eine anti-digoxigenin-antikörper-gekoppelte alkalische Phosphatase.

## Patentansprüche

1. Verfahren zum diagnostischen Nachweis mindestens einer der Erkrankungen aus der Gruppe Niereninsuffizienz, Entzündungen, insbesondere Hepatitis, Pankreatitis, Morbus Crohn oder Glomerulonephritis, Diabetes mellitus, mikrobielle oder virale Infektion, Morbus Alzheimer, Hyperkatabolismus, Lungenfibrose und Leberzirrhose, wobei das Verfahren das Bestimmen des Expressionsgrades der humanen zellvolumenregulierten Kinase h-sgk oder des Expressionsgrades ihres Transkriptes umfasst, und wobei Diagnostizierverfahren ausgenommen sind, die am tierischen oder menschlichen Körper vorgenommen werden.

2. Das Verfahren gemäß Anspruch 1, wobei die Erkrankung Hepatitis, Pankreatitis, Morbus Crohn oder Glomerulonephritis ist.

3. Das Verfahren gemäß Anspruch 1, wobei die Erkrankung Diabetes mellitus ist.

4. Das Verfahren gemäß einem der Ansprüche 1 bis 3, wobei der Expressionsgrad einer Nukleinsäure oder mit einer Nukleinsäure ausgewählt aus der Gruppe bestehend aus
(a) Nukleinsäure, kodierend für die humane zellvolumenregulierte Kinase h-sgk mit der Aminosäuresequenz gemäß SEQ ID NO: 2 oder Nukleinsäure mit der Nukleotidsequenz gemäß SEQ ID NO: 1
(b) Nukleinsäure, die mit der Nukleinsäure gemäß (a) unter stringenten Bedingungen hybridisiert und die für eine funktionell aktive zellvolumenregulierte Kinase kodiert, welche nicht identisch ist mit der Ratten-sgk,
(c) geeignete Probe, Sonde oder Primer einer der unter (a) oder (b) genannten Nukleinsäuren,
bestimmt wird.

5. Das Verfahren gemäß einem der Ansprüche 1 bis 4, wobei der Expressionsgrad mit einem Antikörper bestimmt wird, der spezifisch an die h-sgk bindet.

6. Das Verfahren gemäß einem der Ansprüche 1 bis 5, wobei der Expressionsgrad mit Western blot, Enzyme Linked Immunosorbent Assay (ELISA), Immunhistochemie, Northern blot, oder In-Situ-Hybridisierung bestimmt wird.

7. Verwendung von gegen die h-sgk gerichteten monoklonalen oder polyklonalen Antikörpern zum diagnostischen Nachweis mindestens einer der Erkrankungen aus der Gruppe Niereninsuffizienz, Entzündungen, insbesondere Hepatitis, Pankreatitis, Morbus Crohn oder Glomerulonephritis, Diabetes mellitus, mikrobielle oder virale Infektion, Morbus Alzheimer, Hyperkatabolismus, Lungenfibrose und Leberzirrhose, ausgenommen Diagnostizierverfahren, die am tierischen oder menschlichen Körper vorgenommen werden.

## Claims

1. Method for the diagnostic detection of at least one of the diseases from the group of renal insufficiency, inflammations, in particular hepatitis, pancreatitis, Crohn's disease or glomerulonephritis, diabetes mellitus, microbial or viral infection, Alzheimer's disease, hypercatabolism, pulmonary fibrosis and cirrhosis of the liver, wherein the method comprises determination of the degree of expression of the human cell volume regulated kinase h-sgk or the degree of expression of its transcript, and wherein diagnostic methods which are conducted on the animal or human body are excluded.

2. The method according to claim 1, wherein the disease is hepatitis, pancreatitis, Crohn's disease or glomerulonephritis.

3. The method according to claim 1, wherein the disease is diabetes mellitus.

4. The method according to one of claims 1 to 3, wherein the degree of expression of a nucleic acid or with a nucleic acid chosen from the group consisting of
(a) nucleic acid which codes for the human cell volume regulated kinase h-sgk with the amino acid sequence according to SEQ ID NO: 2 or nucleic acid with the nucleotide sequence according to SEQ ID NO: 1,
(b) nucleic acid which hybridizes with the nucleic acid according to (a) under stringent conditions and which codes for a functionally active cell volume regulated kinase which is not identical to rat sgk,
(c) suitable sample, probe or primer of one of the nucleic acids mentioned under (a) or (b)
is determined.

5. The method according to one of claims 1 to 4, wherein the degree of expression is determined with an antibody which binds specifically to h-sgk.

6. The method according to one of claims 1 to 5, wherein the degree of expression is determined with western blot, enzyme linked immunosorbent assay (ELISA), immunohistochemistry, northern blot or in situ hybridization.

7. Use of monoclonal or polyclonal antibodies directed against h-sgk for the diagnostic detection of at least one of the diseases from the group of renal insufficiency, inflammations, in particular hepatitis, pancreatitis, Crohn's disease or glomerulonephritis, diabetes mellitus, microbial or viral infection, Alzheimer's disease, hypercatabolism, pulmonary fibrosis and cirrhosis of the liver, excluding diagnostic methods which are conducted on the animal or human body.

## Revendications

1. Procédé pour la détection diagnostique d'au moins l'une des maladies du groupe constitué par l'insuffisance rénale, les inflammations, en particulier l'hépatite, la pancréatite, la maladie de Crohn ou la glomérulonéphrite, le diabète sucré, les infections microbiennes ou virales, la maladie d'Alzheimer, l'hypercatabolisme, la fibrose pulmonaire et la cirrhose du foie, le procédé comprenant la détermination du degré d'expression de la kinase h-sgk humaine régulée par le volume cellulaire ou du degré d'expression de son produit de transcription, à l'exclusion des procédés de diagnostic qui sont mis en oeuvre sur le corps animal ou humain.

2. Procédé selon la revendication 1, dans lequel la maladie est l'hépatite, la pancréatite, la maladie de Crohn ou la glomérulonéphrite.

3. Procédé selon la revendication 1, dans lequel la maladie est le diabète sucré.

4. Procédé selon l'une des revendications 1 à 3, dans lequel le degré d'expression est déterminé d'un acide nucléique, ou avec un acide nucléique choisi dans le groupe consistant en
(a) un acide nucléique codant pour la kinase h-sgk humaine régulée par le volume cellulaire, ayant la séquence d'acides aminés selon SÉQ ID N° 2, ou un acide nucléique ayant la séquence nucléotidique selon SÉQ ID N° 1,
(b) un acide nucléique qui est hybridé dans des conditions stringentes avec l'acide nucléique selon (a) et code pour une kinase régulée par le volume cellulaire, fonctionnelle active, qui n'est pas identique à la sgk de rat,
(c) un échantillon, une sonde ou une amorce approprié de l'un des acides nucléiques mentionnés en (a) ou (b).

5. Procédé selon l'une des revendications 1 à 4, dans lequel le degré d'expression est déterminé à l'aide d'un anticorps qui se lie d'une manière spécifique à la h-sgk.

6. Procédé selon l'une des revendications 1 à 5, dans lequel le degré d'expression est déterminé par transfert de Western, analyse par immunosorption à liaison enzymatique (ELISA), immunohistochimie, transfert de Northern ou hybridation in situ.

7. Utilisation d'anticorps monoclonaux ou poly-clonaux dirigés contre la h-sgk pour la détection diagnostique d'au moins l'une des maladies du groupe comprenant l'insuffisance rénale, les inflammations, en particulier l'hépatite, la pancréatite, la maladie de Crohn ou la glomérulonéphrite, le diabète sucré, les infections microbiennes ou virales, la maladie d'Alzheimer, l'hypercatabolisme, la fibrose pulmonaire et la cirrhose du foie, à l'exception des procédés de diagnostic qui sont mis en oeuvre sur le corps animal ou humain.


### SEQUENZPROTOKOLL

(1) ALLGEMEINE ANGABEN:
   (i) ANMELDER:
      (A). NAME: Behring Diagnostics GmbH
      (B) STRASSE: Emil-von-Behring-Str. 76
      (C) ORT: Marburg
      (E) LAND: Germany
      (F) POSTLEITZAHL: 35041
      (G) TELEFON: 06421-39-2332
      (H) TELEFAX: 06421-39-4558
   (ii) BEZEICHNUNG DER ERFINDUNG: Zellvolumenregulierte humane Kinase h-sgk
   (iii) ANZAHL DER SEQUENZEN: 2
   (iv) COMPUTER-LESBARE FASSUNG:
      (A) DATENTRGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LNGE: 2370 Basenpaare
      (B) ART: Nucleotid
      (C) STRANGFORM: Doppelstrang
      (D) TOPOLOGIE: linear
   (ii) ART DES MOLEKLS: Genom-DNA
   (iii) HYPOTHETISCH: NEIN
   (iv) ANTISENSE: NEIN .
   (vi) URSPRNLICHE HERKUNFT:
      (A) ORGANISMUS: Homo sapiens
      (B) STAMM: Caucasian
   (vii) UNMITTELBARE HERKUNFT:
      (B) CLON(E): HSRNASTPK
   (ix) MERKMAL:
      (A) NAME/SCHLSSEL: CDS
      (B) LAGE:43..1335
   (xi) SEQUENZBESCHREIBUNG: SEQ ID NO: 1: 
CACGAGGGAG CGCTAACGTC TTTCTGTCTC CCCGCGGTGG TG ATG ACG GTG AAA 54

1

ACT GAG GCT GCT AAG GGC ACC CTC ACT TAC TCC AGG ATG AGG GGC ATG 102

5                   10                  15                  20

GTG GCA ATT CTC ATC GCT TTC ATG AAG CAG AGG AGG ATG GGT CTG AAC 150

                25                  30                  35
 
GAC TTT ATT CAG AAG ATT GCC AAT AAC TCC TAT GCA TGC AAA CAC CCT 198

            40                  45                  50

GAA GTT CAG TCC ATC TTG AAG ATC TCC CAA CCT CAG GAG CCT GAG CTT 246

        55                  60                  65

ATG AAT GCC AAC CCT TCT CCT CCA CCA AGT CCT TCT CAG CAA ATC AAC 294

    70                  75                  80

CTT GGC CCG TCG TCC AAT CCT CAT GCT AAA CCA TCT GAC TTT CAC TTC 342

85                  90                  95                  100

TTG AAA GTG ATC GGA AAG GGC AGT TTT GGA AAG GTT CTT CTA GCA AGA 390

                105                 110                 115

CAC AAG GCA GAA GAA GTG TTC TAT GCA GTC AAA GTT TTA CAG AAG AAA 438

            120                 125                 130

GCA ATC CTG AAA AAG AAA GAG GAG AAG CAT ATT ATG TCG GAG CGG AAT 486

        135                 140                 145

GTT CTG TTG AAG AAT GTG AAG CAC CCT TTC CTG GTG GGC CTT CAC TTC 534

    150                 155                 160

TCT TTC CAG ACT GCT GAC AAA TTG TAC TTT GTC CTA GAC TAC ATT AAT 582

165                 170                 175                 180

GGT GGA GAG TTG TTC TAC CAT CTC CAG AGG GAA CGC TGC TTC CTG GAA 630

                185                 190                 195

CCA CGG GCT CGT TTC TAT GCT GCT GAA ATA GCC AGT GCC TTG GGC TAC 678

            200                 205                 210

CTG CAT TCA CTG AAC ATC GTT TAT AGA GAC TTA AAA CCA GAG AAT ATT 726

        215                 220                 225

TTG CTA GAT TCA CAG GGA CAC ATT GTC CTT ACT GAT TTC GGA CTC TGC 774

    230                 235                 240

AAG GAG AAC ATT GAA CAC AAC AGC ACA ACA TCC ACC TTC TGT GGC ACG 822

245                 250                 255                 260

CCG GAG TAT CTC GCA CCT GAG GTG CTT CAT AAG CAG CCT TAT GAC AGG 870

                265                 270                 275

ACT GTG GAC TGG TGG TGC CTG GGA GCT GTC TTG TAT GAG ATG CTG TAT 918

            280                 285                 290
 
GGC CTG CCG CCT TTT TAT AGC CGA AAC ACA GGT GAA ATG TAG GAC AAC 966

        295                 300                 305

ATT CTG AAC AAG CCT CTC CAG CTG AAA CCA AAT ATT ACA AAT TCC GCA 1014

    310                 315                 320

AGA CAC CTC CTG GAG GGC CTC CTG CAG AAG GAC AGG ACA AAG CGG CTC 1062

325                 330                 335                 340

GGG GCC AAG GAT GAC TTC ATG GAG ATT AAG AGT CAT GTC TTC TTC TCC 1110

                345                 350                 355

TTA ATT AAC TGG GAT GAT CTC ATT AAT AAG AAG ATT ACT CCC CCT TTT 1158

            360                 365                 370

AAC CCA AAT GTG AGT GGG CCC AAC GAG CTA CGG CAC TTT GAC CCC GAG 1206

        375                 380                 385

TTT ACC GAA GAG CCT GTC CCC AAC TCC ATT GGC AAG TCC CCT GAC AGC 1254

    390                 395                 400

GTC CTC GTC ACA GCC AGC GTC AAG GAA GCT GCC GAG GCT TTC CTA GGC 1302

405                 410                 415                 420

TTT TCC TAT GCG CCT CCC ACG GAC TCT TTC CTC TGAACCCTGT TAGGGCTTGG 1355

                425                 430

TTTTAAAGGA TTTTATGTGT GTTTCCGAAT GTTTTAGTTA GCCTTTTGGT GGAGCCGCCA 1415

GCTGACAGGA CATCTTACAA GAGAATTTGC ACATCTCTGG AAGCTTAGCA ATCTTATTGC 1475

ACACTGTTCG CTGGAATTTT TTGAAGAGCA CATTCTCCTC AGTGAGCTCA TGAGGTTTTC 1535

ATTTTTATTC TTCCTTCCAA CGTGGTGCTA TCTCTGAAAC GAGCGTTAGA GTGCCGCCTT 1595

AGACGGAGGC AGGAGTTTCG TTAGAAAGCG GACCTGTTCT AAAAAAGGTC TCCTGCAGAT 1655

CTGTCTGGGC TGTGATGACG AATATTATGA AATGTGCCTT TTCTGAAGAG ATTGTGTTAG 1715

CTCCAAAGCT TTTCCTATCG CAGTGTTTCA GTTCTTTATT TTCCCTTGTG GATATGCTGT 1775

GTGAACCGTC GTGTGAGTGT GGTATGCCTG ATCACAGATG GATTTTGTTA TAAGCATCAA 1835

TGTGACACTT GCAGGACACT ACAACGTGGG ACATTGTTTG TTTCTTCCAT ATTTGGAAGA 1895

TAAATTTATG TGTAGACTTT TTTGTAAGAT ACGGTTAATA ACTAAAATTT ATTGAAATGG 1955

TCTTGCAATG ACTCGTATTC AGATGCCTAA AGAAAGCATT GCTGCTACAA ATATTTCTAT 2015

TTTTAGAAAG GGTTTTTATG GACCAATGCC CCAGTTGTCA GTCAGAGCCG TTGGTGTTTT 2075

TCATTGTTTA AAATGTCACC TGTAAAATGG GCATTATTTA TGTTTTTTTT TTTGCATTCC 2135

TGATAATTGT ATGTATTGTA TAAAGAACGT CTGTACATTG GGTTATAACA CTAGTATATT 2195
 
TAAACTTACA GGCTTATTTG TAATGTAAAC CACTATTTTA ATGTACTGTA ATTAACATGG 2255

TTATAATACG TACAATCCTT CCCTCATCCC ATCACACAAC TTTTTTTGTG TGTGATAAAC 2315

TGATTTTGGT TTGCAATAAA ACCTTGAAAA ATAAAAAAAA AAAAAAAAAA AAAAA 2370
(2) ANGABEN ZU SEQ ID NO: 2:
   (i) SEQUENZKENNZEICHEN:
      (A) LNGE: 431 Aminosuren
      (B) ART: Aminosure
      (D) TOPOLOGIE: linear
   (ii) ART DES MOLEKLS: Protein
   (xi) SEQUENZBESCHREIBUNG: SEQ ID NO: 2:


1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240



                245                 250                 255


            260                 265                 270


        275                 280                 285


    290                 295                 300


305                 310                 315                 320


                325                 330                 335


            340                 345                 350


        355                 360                 365


    370                 375                 380


385                 390                 395                 400


                405                 410                 415


            420                 425                 430